# EUROPEAN PATENT APPLICATION

(11) **EP 4 170 032 A1**
(43) Date of publication of application: **26.04.2023**
(21) Application number: 21826587.4
(22) Date of filing: 18.06.2021
(51) Int. Cl.: C12N 15/12, G01N 33/15, G01N 33/50, G01N 33/53, G01N 33/68

(54) **METHOD FOR DETERMINING DISEASE CAUSED BY SYNAPTIC DYSFUNCTION OR DISEASE ACCOMPANIED BY SYNAPTIC DYSFUNCTION**

(30) Priority: 19.06.2020 JP 2020106421
(71) Applicant: AlzMed, Inc., Tokyo 113-8485 (JP)
(72) Inventor: SHIRAO, Tomoaki, Tokyo 113-8485 (JP)
(74) Representative: Engelhard, Markus
(86) International application number: PCT/JP2021/023135
(87) International publication number: WO 2021/256550

(57) **Abstract**

The present invention addresses the problem of providing: a determination method that can determine, early and with ease, whether or not a disease caused by synaptic dysfunction or a disease accompanied by synaptic dysfunction has occurred, and the severity level of the disease; and a screening method for a therapeutic agent and a prophylactic agent for a disease caused by synaptic dysfunction or a disease accompanied by synaptic dysfunction. The present invention provides a determination method that can determine disease caused by synaptic dysfunction or a disease accompanied by synaptic dysfunction early and with ease and also can contribute to drug discovery research for these diseases, with a determination method for determining whether or not a disease caused by synaptic dysfunction or a disease accompanied by synaptic dysfunction has occurred, where drebrin A-related proteins (DARPs) serve as indices, and with a screening method for screening a therapeutic agent and prophylactic agent for a disease caused by synaptic dysfunction or a disease accompanied by synaptic dysfunction, where drebrin A-related proteins (DARPs) serve as indices.

## Description

### [Technical Field]

The present invention relates to a determination method that is capable of determining the presence or absence of onset, or severity, of a disease caused by synaptic dysfunction or disease accompanied by synaptic dysfunction in the early stage in a simple manner, a method of screening for a prophylactic agent and/or therapeutic agent for a disease caused by synaptic dysfunction or disease accompanied by synaptic dysfunction, an antibody for use in said determination method or screening method, and a kit for carrying out the determination method.

### [Background Art]

Alzheimer's disease, which is the primary cause of dementia, is an irreversible progressive brain disease that is understood be result from dysfunction of a synapse in the brain. The increase in patients of Alzheimer's disease has become a major societal concern in recent years. Establishment of an early diagnosis method or a therapeutic drug thereof is desired. Neuritic plaques are observed in the postmortem brain of Alzheimer's disease patients, which are known as aggregations of "amyloid β protein" (amyloid plaques). Deposition of amyloid β proteins is the earliest lesion that can be pathologically confirmed. It is reported that amyloid β proteins aggregate and directly exhibit neurocytotoxicity. Currently, it is widely accepted that abnormal amyloid β protein production and accumulation is broadly associated with the onset of Alzheimer's disease in view of genetic analysis of familial Alzheimer's disease patients. This is known as the amyloid cascade hypothesis. In this manner, it is widely accepted that amyloid β proteins are the primary cause of Alzheimer's disease in view of numerous studies (Non Patent Literature 1). It is also known that amyloid β protein accumulation in the brain starts 20 year or more before the onset.

It is known that synaptic dysfunction is caused due to reduced blood flow in the brain (depression, etc.), cerebral hemorrhage, cerebral infarction, etc. in addition to Alzheimer's disease. Dementia due to a mitochondrial genetic disease or diabetes, etc. is also known. Synaptic dysfunction may also occur due to drug dependence, etc. While it is understood that the cognitive function deteriorates due to synaptic dysfunction in a disease caused by such synaptic dysfunction or disease accompanied by such synaptic dysfunction, but the diagnosis thereof is challenging. For example, the diagnosis of Alzheimer's disease can be confirmed only through post-mortem pathological autopsy. Although Alzheimer's disease is diagnosed through a Q&A test or image test for diagnosis while the patient is alive, the reality is that such diagnostic methods are all diagnosis after the manifestation of a subjective symptom, so that therapy cannot be commenced in the early stages. It is also reported that Alzheimer's disease can be diagnosed from a decrease in the concentration of amyloid β42 protein in the cerebrospinal fluid (Non Patent Literature 2), but this is a highly invasive test that has a problem of being difficult to apply to elderly patients. Further, currently used therapeutic drugs for Alzheimer's disease are all drugs that suppress symptoms. There is no pharmaceutical product that leads to fundamental treatment. Other diseases caused by synaptic dysfunction and diseases accompanied by synaptic dysfunction are also dependent on a Q&A test (depression, dementia, etc.) or image test (cerebral hemorrhage, etc.) with no simple method for diagnosis in the early stages.

The inventors were the first in the world to discover the actin-binding protein drebrin, which is highly expressed in neurons during the developmental process (see, for example, Non Patent Literatures 3 and 4). The inventors have already proven that: drebrin is associated with morphogenesis, especially neurite formation, in neurons by changing the attribute of actin fibers (see, for example, Non Patent Literatures 5 to 7); and drebrin is present throughout the soma and neurite in migrating neurons during development, but is present specifically in the spinal structure (dendritic spine) in mature neurons (see, for example, Non Patent Literatures 8 to 10). There are two isoforms of drebrin, i.e., embryonic type drebrin E and adult type drebrin A (see, for example, Non Patent Literature 4). Drebrin A found specifically in the dendritic spine of mature neurons has a characteristic of being expressed in only neurons (see, for example, Non Patent Literatures 9 and 10). The inventors have also reported that drebrin in dendritic spines is eliminated in an extended range in dementia such as Alzheimer's disease (Non Patent Literature 11).

### [Citation List]

### [Non Patent Literature]

[NPL 1] Takashi Saito, Takaomi Nishimichi "Arutsuhaimabyo no Moderu Mausu [mouse Alzheimer's disease model]", Folia Pharmacol. Jpn. 144, 250-252, 2014
[NPL 2] J. Neurol. Sci. 148, 41-45, 1997
[NPL 3] J. Neurochem. 44, 1210-1216, 1985
[NPL 4] J. Biochem. 117, 231-236, 1995
[NPL 5] J. Neurosci. Res. 38, 149-159, 1994
[NPL 6] Exp. Cell Res. 215, 145-153, 1994
[NPL 7] J. Biol. Chem. 269, 29928-29933, 1994
[NPL 8] J. Neurosci. 15, 7161-7170, 1996
[NPL 9] Dev. Brain Res. 29, 233-244, 1986
[NPL 10] Brain Res. 413, 374-378, 1987
[NPL 11] J Neurosci. Res. 43(1), 87-92, 1996

### [Summary of Invention]

### [Technical Problem]

Currently, diseases caused by synaptic dysfunction and diseases accompanied by synaptic dysfunction cannot be determined in the early stages in a simple manner, as described above. Furthermore, synaptic dysfunction cannot be directly treated. For example, Alzheimer's disease cannot be fundamentally treated, so that the treatment thereof is currently symptomatic treatment. The problem to be solved by the present invention is to provide a determination method that is capable of determining the presence or absence of onset, or severity, of a disease caused by synaptic dysfunction or disease accompanied by synaptic dysfunction in the early stage in a simple manner, and to provide a method of screening for a prophylactic agent or therapeutic agent for a disease caused by synaptic dysfunction or disease accompanied by synaptic dysfunction.

### [Solution to Problem]

The inventors contemplated that drebrin A is leaking into the blood from neurons in Alzheimer's disease patients based on the finding that drebrin A in dendritic spines is eliminated in an extended range in dementia such as Alzheimer's disease. In this regard, to detect drebrin A in the blood, protein A/G beads treatment was applied to remove antibodies in the blood in accordance with a common method, and the sample was then subjected to Western blot by using an anti-drebrin antibody that recognizes drebrin A and drebrin E, but a band of drebrin could not be found. When the inventors subjected a fraction precipitated through protein A/G beads of a blood sample to Western blot by using an anti-drebrin antibody, a band of drebrin was unexpectedly found at a location corresponding to about 130 kDa. When a fraction precipitated through protein A/G beads of a blood sample was subjected to Western blot using an anti-drebrin A antibody to check whether the band of drebrin is drebrin A, the band at about 130 kDa was drebrin E, not full length drebrin A, but a plurality of bands with a smaller molecular weight than full length drebrin A, which can be detected with an anti-drebrin A antibody, were able to be detected. The bands detected are understood to be fragments or splice variants of drebrin A having a translation peptide of an Ins2 sequence which is characteristic in drebrin A genes. The inventors have also discovered after further studies that such drebrin A related proteins (DARPs) are present in a large quantity in a biological sample derived from patients suffering from a disease caused by synaptic dysfunction or disease accompanied by synaptic dysfunction. The inventors also discovered that: DARPs are bound to protein A/G beads via an autoantibody; an autoantibody that recognizes drebrin can damage neurons; diseases caused by synaptic dysfunction and diseases accompanied by synaptic dysfunction can be treated or prevented by suppressing damage to neurons by an autoantibody. The present invention was completed through such findings.

Specifically, the present invention relates to the following.
[1] A method of determining a risk of onset, or the presence or absence of onset of, a disease caused by synaptic dysfunction or disease accompanied by synaptic dysfunction, comprising steps (a-1) to (c-1):
   (a-1) a step for measuring a concentration of drebrin A related proteins (DARPs) in a biological sample harvested from a subject;
   (b-1) a step for comparing the concentration of DARPs measured in step (a-1) with a concentration of DARPs in a control who does not develop a disease caused by synaptic dysfunction or disease accompanied by synaptic dysfunction; and
   (c-1) a step for evaluating the subject as having a high risk of onset of a disease caused by synaptic dysfunction or disease accompanied by synaptic dysfunction or as highly likely to have developed a disease caused by synaptic dysfunction or disease accompanied by synaptic dysfunction if the concentration of DARPs measured in step (a-1) is higher than the concentration of DARPs in the control.
[2] A method of determining a severity of a disease caused by synaptic dysfunction or disease accompanied by synaptic dysfunction, comprising steps (a-2) to (c-2):
   (a-2) a step for measuring a concentration of drebrin A related proteins (DARPs) in a biological sample harvested from a subject who has developed a disease caused by synaptic dysfunction or disease accompanied by synaptic dysfunction;
   (b-2) a step for comparing the concentration of DARPs measured in step (a-2) with a concentration of DARPs in a control who has developed a disease caused by synaptic dysfunction or disease accompanied by synaptic dysfunction; and
   (c-2) a step for evaluating the subject as highly likely to have a more severe disease caused by synaptic dysfunction or disease accompanied by synaptic dysfunction than the control if the concentration of DARPs and/or DARP autoantibodies measured in step (a-2) is higher than the concentration of DARPs and/or DARP autoantibodies in the control.
[3] A method of screening for a prophylactic agent or a therapeutic agent for a disease caused by synaptic dysfunction or disease accompanied by synaptic dysfunction, comprising steps (a-3) to (c-3):
   (a-3) a step for measuring a concentration of drebrin A related proteins (DARPs) in a sample harvested from a non-human animal synaptic dysfunction model administered with a test substance;
   (b-3) a step for comparing the concentration of drebrin A related proteins (DARPs) measured in step (a-3) with a concentration of drebrin A related proteins (DARPs) in a non-human animal synaptic dysfunction model which is not administered with the test substance; and
   (c-3) a step for evaluating the test substance as effective in prophylaxis or treatment of a disease caused by synaptic dysfunction or disease accompanied by synaptic dysfunction if the concentration of drebrin A related proteins (DARPs) measured in step (a-3) is lower than the concentration of drebrin A related proteins (DARPs) in the non-human animal synaptic dysfunction model which is not administered with the test substance.
[4] A method of screening for a prophylactic agent or a therapeutic agent for a disease caused by synaptic dysfunction or disease accompanied by synaptic dysfunction, comprising steps (a-4) to (d-4):
   (a-4) a step for administering a test substance to a cultured cell synaptic dysfunction model and culturing the model;
   (b-4) a step for measuring a concentration of drebrin A related proteins (DARPs) in a culture of the cultured cell synaptic dysfunction model;
   (c-4) a step for comparing the concentration of DARPs measured in step (b-4) with a concentration of DARPs in a culture of a cultured cell synaptic dysfunction model which is not administered with the test substance; and
   (d-4) a step for evaluating the test substance as effective in prophylaxis or treatment of a disease caused by synaptic dysfunction or disease accompanied by synaptic dysfunction if the concentration of DARPs measured in step (b-4) is lower than the concentration of DARPs in the culture of a cultured cell synaptic dysfunction model which is not administered
   with the test substance.
[5] The method of [1] to [3], wherein the biological sample is a cerebrospinal fluid sample or a blood sample.
[6] The method of [1] to [5], wherein the DARPs are one or more types selected from DARP40, DARP60, DARP70, DARP90, and DARP100.
[7] The method of [1] to [6], wherein the disease caused by synaptic dysfunction or disease accompanied by synaptic dysfunction is a neurodegenerative disease.
[8] The method of [7], wherein the neurodegenerative disease is selected from Alzheimer's disease, corticobasal syndrome, Parkinson's disease, spinocerebellar degeneration, and amyotrophic lateral sclerosis.
[9] The method of [1] to [8], wherein the concentration of DARPs is measured by using an antibody that recognizes a drebrin A specific epitope.
[10] An antibody for use in the method of [9], characterized by recognizing a drebrin A specific epitope.
[11] A kit for determining a risk of onset, the presence or absence of onset, or severity of a disease caused by synaptic dysfunction or disease accompanied by synaptic dysfunction, comprising the antibody of [10].
[12] A method of screening for a prophylactic agent or a therapeutic agent for a disease caused by synaptic dysfunction or disease accompanied by synaptic dysfunction, characterized by searching for a dominant negative peptide to a drebrin A related protein (DARP) autoantibody.

Other embodiments of the present invention include:
a method of determining a risk of onset, or the presence or absence of onset of, a disease caused by synaptic dysfunction or disease accompanied by synaptic dysfunction, comprising: step (a-1) for measuring a concentration of drebrin A related proteins (DARPs) in a biological sample harvested from a subject;
a method of measuring a severity of a disease caused by synaptic dysfunction or disease accompanied by synaptic dysfunction, comprising: step (a-2) for measuring a concentration of drebrin A related proteins (DARPs) in a biological sample harvested from a subject who has developed a disease caused by synaptic dysfunction or disease accompanied by synaptic dysfunction;
a biomarker for determining a risk of onset, or the presence or absence of onset of, a disease caused by synaptic dysfunction or disease accompanied by synaptic dysfunction, or for determining a severity of a disease caused by synaptic dysfunction or disease accompanied by synaptic dysfunction, consisting of one or more drebrin A related proteins (DARPs);
a method of screening for a prophylactic agent or a therapeutic agent for a disease caused by synaptic dysfunction or disease accompanied by synaptic dysfunction, comprising: step (a-3) for measuring a concentration of drebrin A related proteins (DARPs) in a sample harvested from a non-human animal synaptic dysfunction model administered with a test substance;
a method of screening for a prophylactic agent or a therapeutic agent for a disease caused by synaptic dysfunction or disease accompanied by synaptic dysfunction, comprising: steps (a-4) and (b-4);
a method of collecting data for diagnosing a risk of onset, or the presence or absence of onset of, a disease caused by synaptic dysfunction or disease accompanied by synaptic dysfunction, comprising: steps (a-1) to (c-1);
a method of collecting data for diagnosing a severity of a disease caused by synaptic dysfunction or disease accompanied by synaptic dysfunction, comprising: steps (a-2) to (c-2);
a method of determining efficacy of a prophylactic agent or a therapeutic agent for a disease caused by synaptic dysfunction or disease accompanied by synaptic dysfunction, comprising: steps (a-3) to (c-3);
a method of determining efficacy of a prophylactic agent or a therapeutic agent for a disease caused by synaptic dysfunction or disease accompanied by synaptic dysfunction, comprising: steps (a-4) to (d-4); and
a prophylactic agent or a therapeutic agent for a disease caused by synaptic dysfunction or disease accompanied by synaptic dysfunction, comprising a dominant negative peptide to a drebrin A related protein (DARP) autoantibody.

### [Advantageous Effects of Invention]

Since the presence or absence of onset or severity of a disease caused by synaptic dysfunction or disease accompanied by synaptic dysfunction, which depended on a Q&A test (depression, dementia, etc.) or image test (cerebral hemorrhage, etc.) in the past, can be determined in the early stages in a simple manner with the determination method of the invention, such a method leads to prevention of onset of such diseases and to treatment in the early stages. Furthermore, DARPs and anti-DARP autoantibodies can be an indicator for screening for a prophylactic agent or therapeutic agent and contribute to research for drug discovery.

### [Brief Description of Drawings]

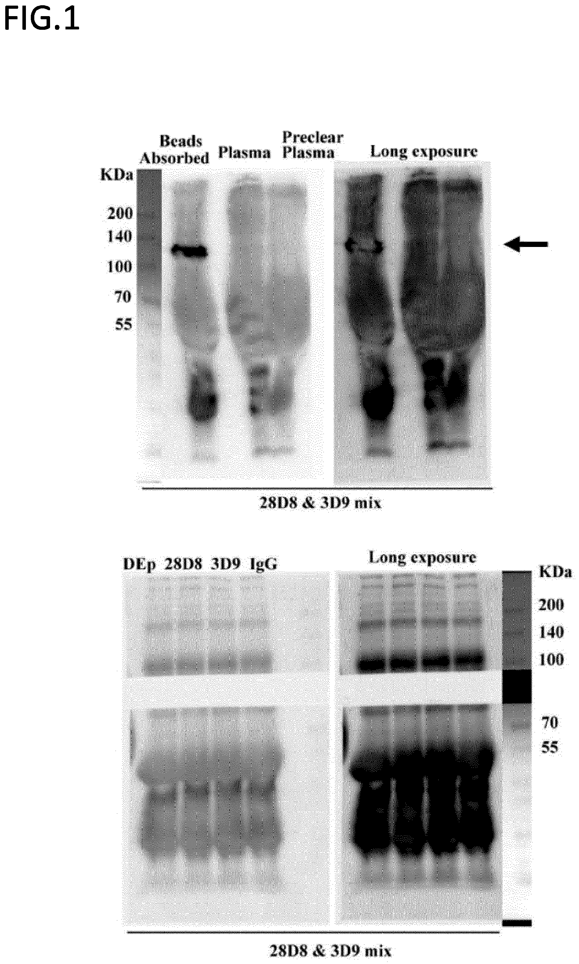

[Figure 1] The top row is a diagram showing results of analyzing beads absorbed fraction (Beads Absorbed), plasma (Plasma), and preclear plasma (Preclear Plasma) by Western blot in Example 1. The bottom row is a diagram showing results of analyzing each IP fraction by Western blot in Example 1.

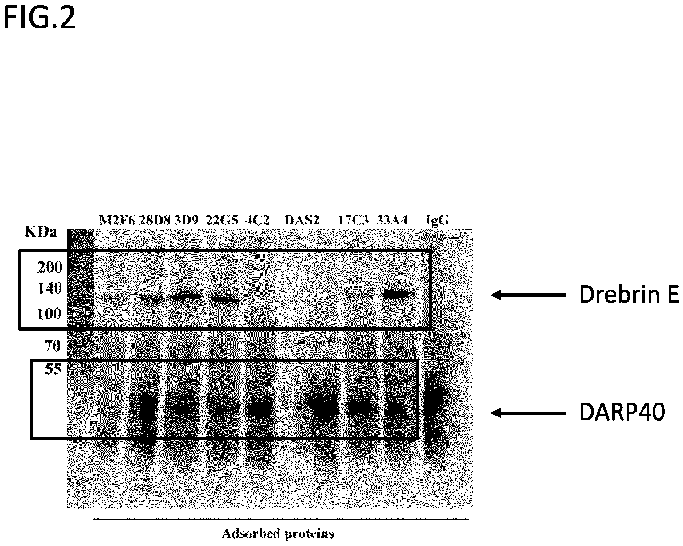

[Figure 2] Figure **2** is a diagram showing results of Western blot using various anti-drebrin antibodies (M2F6, 28D8, 3D9, 22G5, 17C3, 33A4: prepared by the inventors) and anti-drebrin A antibodies (4C2: prepared by the inventors; DAS2: Immuno-Biological Laboratories) on the beads absorbed fraction in Example 1.

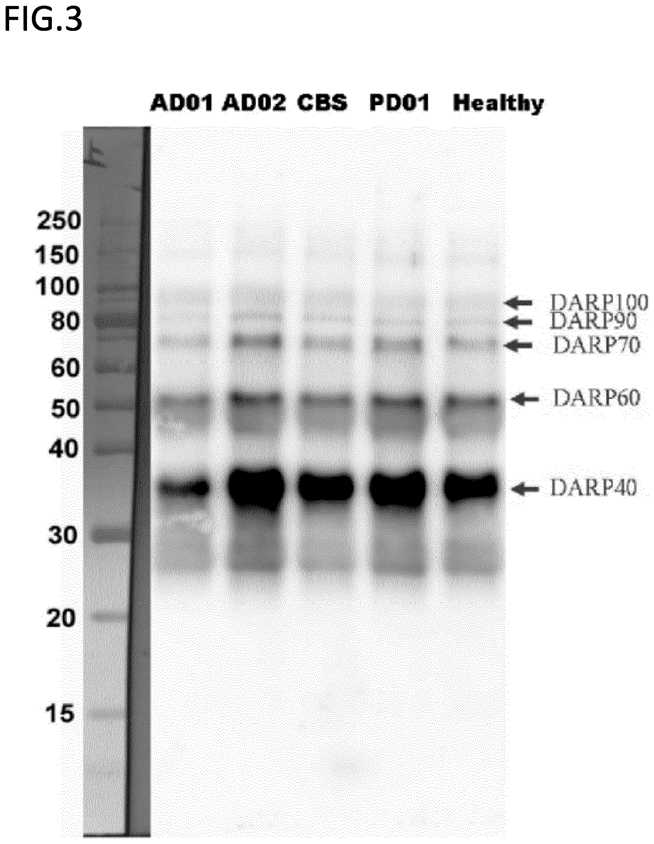

[Figure 3] Figure **3** is a diagram showing results of obtaining a blood sample from two Alzheimer's disease patients (ADO1, ADO2), one corticobasal syndrome patient (CBS), one Parkinson's disease patient (PD01), and a healthy 65 year old male (Healthy) and performing Western blot using an anti-drebrin A antibody DAS2 (Immuno-Biological Laboratories) on a protein A/G beads absorbed fraction in Example 2.

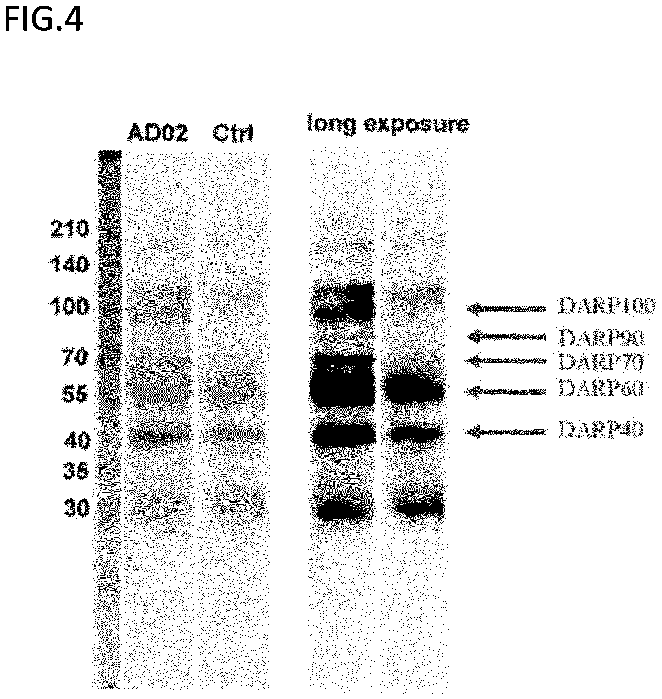

[Figure 4] Figure **4** is a diagram showing results of detecting DARPs through Western blot by using AD02 and control plasma (Ctrl) in Example 2. The concentration of each of the DARPs, i.e., DARP40, DARP60, DARP70, DARP90, and DARP100, was elevated in AD02.

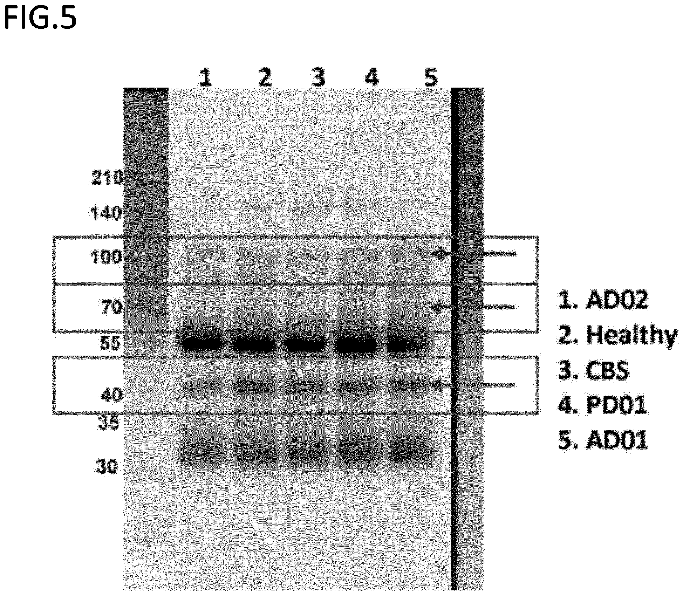

[Figure 5] Figure **5** is a diagram showing results of obtaining a blood sample from two Alzheimer's disease patients (ADO1, ADO2), one corticobasal syndrome patient (CBS), one Parkinson's disease patient (PD01), and a healthy 65 year old male (Healthy) and performing Western blot using an anti-drebrin antibody 3D9 (prepared by the inventors) on a protein A/G beads absorbed fraction in Example 2. Unlike Figure **3****,** a difference between samples was not found.

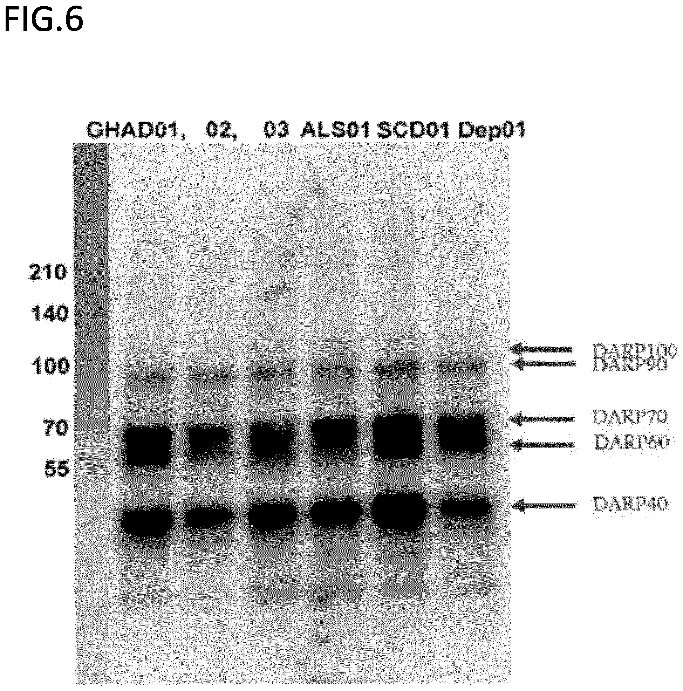

[Figure 6] Figure **6** is a diagram showing results of obtaining a cerebrospinal fluid sample from three Alzheimer's disease patients (GHAD01 to 03, each having mild dementia), one amyotrophic lateral sclerosis patient (ALS01), one spinocerebellar degeneration patient (SCD01), and one depression patient (Dep01) and performing Western blot using an anti-drebrin A antibody DAS2 (Immuno-Biological Laboratories) on a protein A/G beads absorbed fraction in Example 3.

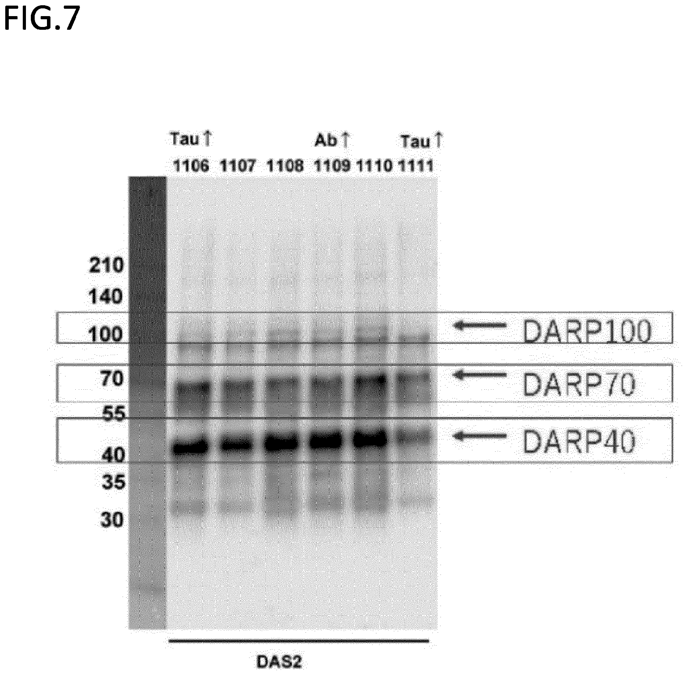

[Figure 7] Figure **7** is a diagram showing results of detecting DARPs in a cerebrospinal fluid sample through Western blot for six Alzheimer's disease patients described in Table 1 in Example 4.

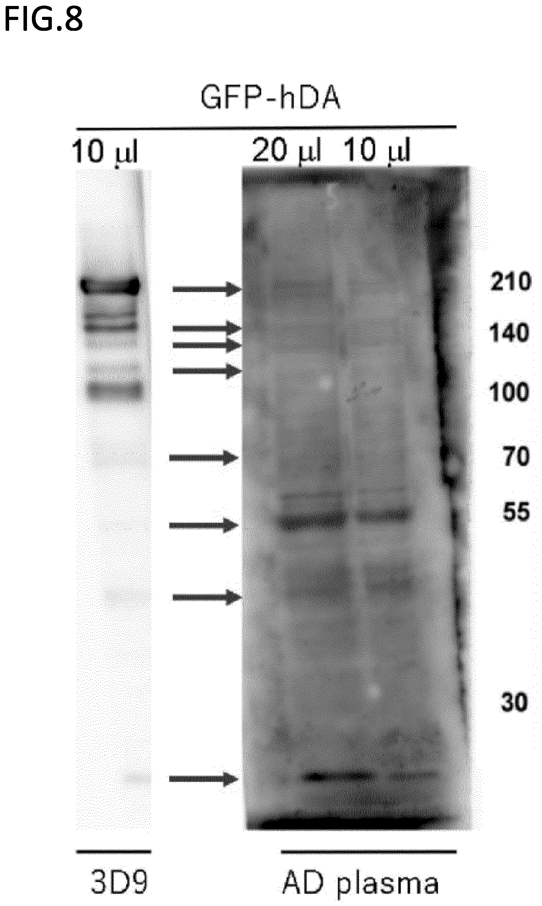

[Figure 8] Figure **8** is a diagram showing results of subjecting GFP tag added human drebrin A (GFP-hDA) to SDS-PAGE and detection through Western blot using an anti-drebrin antibody (3D9: prepared by the inventors) (left) and Alzheimer's disease patient derived plasma (AD plasma) (right) as a primary antibody in Example 5.

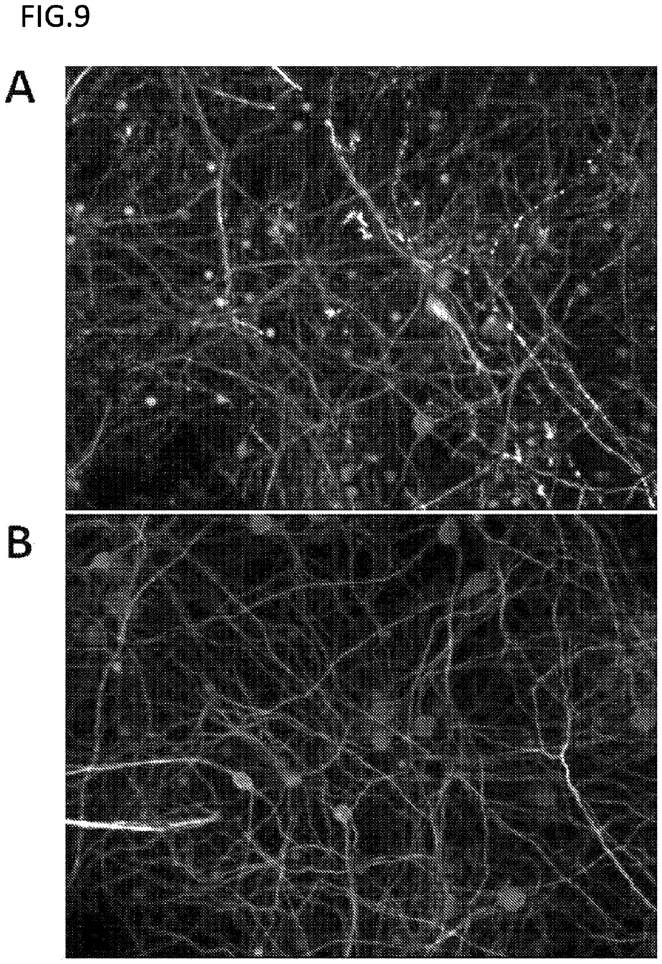

[Figure 9] Figure 9 is a diagram showing results of checking whether an anti-drebrin antibody (22G5: prepared by the inventors) can be toxic to rat hippocampal cultured cells in Example 5. (A) shows a rat hippocampal cultured cell subjected to 22G5 treatment, and (B) shows a rat hippocampal cultured cell subjected to normal mouse IgG treatment.

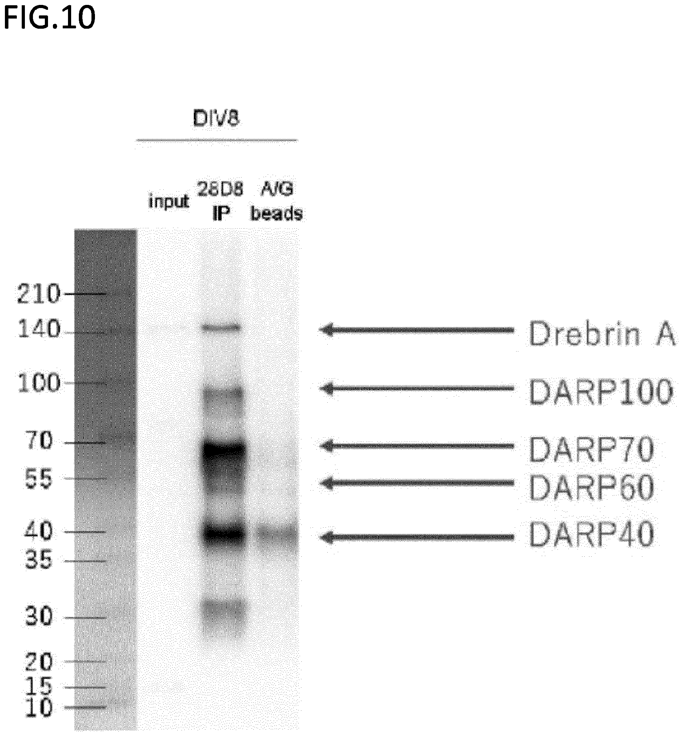

[Figure 10] Figure **10** is a diagram showing results of Western blot for confirming the presence of DARPs in a cultured rat cerebral cortical cell in Example 6.

### [Description of Embodiments]

The present invention is not particularly limited, as long as it is a method of determining a risk of onset, or the presence or absence of onset of, a disease caused by synaptic dysfunction or disease accompanied by synaptic dysfunction, comprising: (a-1) a step for measuring a concentration of drebrin A related proteins (DARPs) in a biological sample harvested from a subject; (b-1) a step for comparing the concentration of DARPs measured in step (a-1) with a concentration of DARPs in a control who does not develop a disease caused by synaptic dysfunction or disease accompanied by synaptic dysfunction; and (c-1) a step for evaluating the subject as having a high risk of onset of a disease caused by synaptic dysfunction or disease accompanied by synaptic dysfunction or as highly likely to have developed a disease caused by synaptic dysfunction or disease accompanied by synaptic dysfunction if the concentration of DARPs measured in step (a-1) is higher than the concentration of DARPs in the control (hereinafter, also referred to as "present determination method 1"), or
a method of determining a severity of a disease caused by synaptic dysfunction or disease accompanied by synaptic dysfunction, comprising: (a-2) a step for measuring a concentration of drebrin A related proteins (DARPs) in a biological sample harvested from a subject who has developed a disease caused by synaptic dysfunction or disease accompanied by synaptic dysfunction; (b-2) a step for comparing the concentration of DARPs measured in step (a-2) with a concentration of DARPs in a control who has developed a disease caused by synaptic dysfunction or disease accompanied by synaptic dysfunction; and (c-2) a step for evaluating the subject as highly likely to have a more severe disease caused by synaptic dysfunction or disease accompanied by synaptic dysfunction than the control if the concentration of DARPs and/or DARP autoantibodies measured in step (a-2) is higher than the concentration of DARPs and/or DARP autoantibodies in the control (hereinafter, also referred to as "present determination method 2"; hereinafter, present determination method 1 and present determination method 2 may also be collectively referred to as " present determination methods"). In this regard, "severity" includes an acute exacerbation stage in which a synaptic disorder advances rapidly. In one embodiment, the present determination methods are methods of assisting diagnosis of a risk of onset, or diagnosis of the presence or absence of onset or severity of, a disease caused by synaptic dysfunction or disease accompanied by synaptic dysfunction by a physician, wherein the method may not comprise an act of diagnosis by a physician.

The present invention is not particularly limited, as long as it is a method of screening for a prophylactic agent or a therapeutic agent for a disease caused by synaptic dysfunction or disease accompanied by synaptic dysfunction, comprising: (a-3) a step for measuring a concentration of drebrin A related proteins (DARPs) in a sample harvested from a non-human animal synaptic dysfunction model administered with a test substance; (b-3) a step for comparing the concentration of drebrin A related proteins (DARPs) measured in step (a-3) with a concentration of drebrin A related proteins (DARPs) in a non-human animal synaptic dysfunction model which is not administered with the test substance; and (c-3) a step for evaluating the test substance as effective in prophylaxis or treatment of a disease caused by synaptic dysfunction or disease accompanied by synaptic dysfunction if the concentration of drebrin A related proteins (DARPs) measured in step (a-3) is lower than the concentration of drebrin A related proteins (DARPs) in the non-human animal synaptic dysfunction model which is not administered with the test substance (hereinafter, also referred to as "instant screening method 1"), or
a method of screening for a prophylactic agent or a therapeutic agent for a disease caused by synaptic dysfunction or disease accompanied by synaptic dysfunction, comprising: (a-4) a step for administering a test substance to a cultured cell synaptic dysfunction model and culturing the model; (b-4) a step for measuring a concentration of drebrin A related proteins (DARPs) in a culture of the cultured cell synaptic dysfunction model; (c-4) a step for comparing the concentration of DARPs measured in step (b-4) with a concentration of DARPs in a culture of a cultured cell synaptic dysfunction model which is not administered with the test substance; and (d-4) a step for evaluating the test substance as effective in prophylaxis or treatment of a disease caused by synaptic dysfunction or disease accompanied by synaptic dysfunction if the concentration of DARPs measured in step (b-4) is lower than the concentration of DARPs in the culture of a cultured cell synaptic dysfunction model which is not administered with the test substance (hereinafter, also referred to as "instant screening method 2"; hereinafter, instant screening method 1 and instant screening method 2 may also be collectively referred to as "instant screening methods").

The present invention was completed by discovering for the first time that many drebrin A related proteins (DARPs), which are fragments or splice variants of human drebrin A (SEQ ID NO: 1) that are localized in the brain, are present in a biological sample derived from a patient suffering from a disease caused by synaptic dysfunction or disease accompanied by synaptic dysfunction. As used herein, DARPs may be any fragment or splice variant of drebrin A having at least a portion of Ins2 translation sequence RPYCPFIKASDSGPSSSSSSSSSPPRTPFPYITCHRTPNLSSSLPC (SEQ ID NO: 2), which is characteristic to drebrin A. Preferred examples thereof include DARP100 with a molecular weight of about 100 kDa, DARP90 with a molecular weight of about 90 kDa, DARP70 with a molecular weight of about 70 kDa, DARP60 with a molecular weight of about 60 kDa, and DARP40 with a molecular weight of about 40 kDa. DARPs can also be used in the present determination methods or instant screening methods, independently or as a combination of one or more DARPs. The total concentration of DARPs can also be measured and used in the instant determinations methods or instant screening methods.

The present invention can measure the concentration of DARPs by any known method. In a preferred embodiment, the concentration of DARPs is measured by Western blot, ELISA (Enzyme-Linked Immuno Sorbent Assay), immune precipitation, etc. using an antibody that recognizes DARPs (hereinafter, also referred to as "anti-DARP antibody"). An anti-DARP antibody may be a monoclonal antibody or a polyclonal antibody, as long as it is an antibody having a part of DARPs as an epitope. One type or a combination of two or more types thereof may be used. It is preferable to measure the concentration by using an antibody that recognizes a drebrin A specific epitope in order to distinguish drebrin A from drebrin E that is present throughout the entire body. In one embodiment, the present invention relates to an antibody that recognizes a drebrin A specific epitope for use in the present determination methods and/or instant screening methods (hereinafter, also referred to as "instant antibody"). In this regard, "drebrin A specific epitope" refers to an epitope that is present in drebrin A, but not in drebrin E, and is preferably a region comprising at least a part of Ins2 translation sequence RPYCPFIKASDSGPSSSSSSSSSPPRTPFPYITCHRTPNLSSSLPC (SEQ ID NO: 2) and/or a three-dimensional structure specifically made so that drebrin A includes the Ins2 translation sequence.

When performing Western blot or ELISA, it is preferable to use an anti-DARP antibody as a primary antibody, and a method of detection using a labeled secondary antibody that recognizes the primary antibody. For example, if the primary antibody is a rabbit antibody, a labeled anti-rabbit IgG antibody can be used, and if the primary antibody is a mouse antibody, a labeled anti-mouse IgG antibody can be used as a secondary antibody.

Examples of a labeling substance in the labeled secondary antibody described above include enzymes, radioisotopes, fluorescent substances, luminescent substances, gold colloids, etc. Among them, an enzyme is preferable, and horseradish peroxidase (HRP), alkaline phosphatase (AP), glucose oxidase (GOD), etc. are more preferable from the viewpoint of sensitivity and ease of handling. If HRP is used as a labeling substance, TMB (3,3',5,5'-tetramethylbenzidine), etc. can be used as a substrate, and if AP is used, AMPPD (3-(2'-spiroadamantane)-4-methoxy-4-(3''-phosphoryloxy)phenyl-1,2-dioxetane disodium salt), 9-(4-chlorophenylthiophosphoryloxymethylidene)-10-methylacridane disodium salt, etc. can be used as a substrate. As a labeling substance, fluorescent dyes such as FITC (fluorescein isothiocyanate) or rhodamine can also be used.

The method of detecting/quantifying DARPs varies depending on the labeling method and can be performed by a method that is well known and conventional to those skilled in the art. For example, if HRP, AP, GOD, etc. is used as a labeling substance, a substance to be measured can be quantified by adding a chromogenic substance or luminescent substance and measuring the absorbance or luminescence intensity. If a fluorescent substance is used as a labeling substance, a substance to be measured can be quantified by measuring the fluorescence intensity thereof. If a radioisotope is used as a labeling substance, a substance to be measured can be quantified by measuring radiation. If a gold colloid is used as a labeling substance, a substance to be measured can be quantified by measuring the absorbance. Quantification may involve, for example, creating calibration curves (standard curves) in advance with a sample having a known concentration of DARPs, collating measurement values with the calibration curves to calculate the concentration of DARPs in a sample, and using the concentration for comparison in the present determination methods or instant screening methods, but absorbance, radiation, or luminescence intensity may be directly used for comparison in the present determination methods or instant screening methods without calculating the concentration of DARPs. In this regard, any threshold value (cut-off value) can be set to evaluate (determine) whether the concentration of DARPs in a subject (the concentration of DARPs calculated above, or absorbance, radiation, or luminescence intensity) is higher or lower than the concentration of DARPs in a control (the concentration of DARPs calculated above, or absorbance, radiation, or luminescence intensity) in the present determination methods. Examples of such a threshold value include the mean, mean + standard deviation (SD), mean + 2 SD, mean + 3 SD, mean - SD, mean - 2 SD, mean - 3 SD, median, median + SD, median + 2 SD, median + 3 SD, median - SD, median - 2 SD, median - 3 SD, etc. of the concentrations of DARPs in the control. Further, any threshold value (cut-off value) can be set to evaluate (determine) whether the concentration of DARPs in a non-human animal synaptic dysfunction model or cultured cell synaptic dysfunction model administered with a test substance (the concentration of DARPs calculated above, or absorbance, radiation, or luminescence intensity) is higher or lower than the concentration of DARPs in a non-human animal synaptic dysfunction model or cultured cell synaptic dysfunction model not administered with the test substance (the concentration of DARPs calculated above, or absorbance, radiation, or luminescence intensity) in the instant screening methods. Examples of such a threshold value include the mean, mean + standard deviation (SD), mean + 2 SD, mean + 3 SD, mean - SD, mean - 2 SD, mean - 3 SD, median, median + SD, median + 2 SD, median + 3 SD, median - SD, median - 2 SD, median - 3 SD, etc. of the concentrations of DARPs in the non-human animal synaptic dysfunction model or cultured cell synaptic dysfunction model not administered with a test substance.

As used herein, a biological sample refers to a bodily fluid harvested from a subject or control. In this regard, examples of a bodily fluid include blood, lymph, interstitial fluid, coelomic fluid, cerebrospinal fluid, etc. In particular, cerebrospinal fluid or blood is preferable. Blood contains serum, plasma, etc. While a biological sample may be directly subjected to measurement of a concentration of DARPs, a biological sample is preferably pre-treated to enhance the detection sensitivity of DARPs prior to measurement. A method of pre-treatment may be any method of enhancing the detection sensitivity of a protein subjected to measurement in a biological sample, such as immunoprecipitation using an anti-DARP antibody. The inventors have successfully enhanced the detection sensitivity of DARPs by treating a biological sample with protein A/G beads and subjecting a beads absorbed fraction to the present determination methods or instant screening methods. Protein A/G bead treatment is intended for reducing the background due to an autoantibody prior to immunoprecipitation. A protein of interest is usually detected from a flow-through fraction after protein A/G bead treatment. The discovery that DARPs adsorb onto protein A/G beads by the inventors is not only an unexpected discovery, but also suggests the presence of an anti-DARP autoantibody in a biological sample. The inventors confirmed the presence of an anti-DARP autoantibody in a biological sample, and discovered that an anti-DARP autoantibody can damage brain neurons through additional studies. These discoveries indicate that a dominant negative peptide to an anti-DARP autoantibody can suppress damage of brain neurons due to an anti-DARP autoantibody to treat and/or prevent a disease caused by synaptic dysfunction or disease accompanied by synaptic dysfunction. Thus, in one embodiment, the present invention relates to a method of screening for a prophylactic agent or a therapeutic agent for a disease caused by synaptic dysfunction or disease accompanied by synaptic dysfunction, characterized by searching for a dominant negative peptide to an anti-DARP autoantibody.

As used herein, a disease caused by synaptic dysfunction or disease accompanied by synaptic dysfunction refers to a disease that develops due to synaptic dysfunction, i.e., signaling disorder in the synapse (Alzheimer's disease, cerebral vascular dementia, Lewy body dementia, Parkinson's disease, corticobasal degeneration, amyotrophic lateral sclerosis, spinocerebellar degeneration, etc.) or a disease in which synaptic dysfunction develops after onset of the disease (cerebral hemorrhage, cerebral infarction, brain tumor, etc.). It is known that depression, drug dependence, mitochondrial genetic disease, diabetes, etc. that develop due to reduced blood flow in the brain are also accompanied by synaptic dysfunction and reduce the cognitive function, and are encompassed by a disease caused by synaptic dysfunction or disease accompanied by synaptic dysfunction. The present determination methods can also be used in evaluating an aftereffect in case of a trauma to the brain. A disease caused by synaptic dysfunction or disease accompanied by synaptic dysfunction preferably refers to a neurodegenerative disease, particularly preferably a neurodegenerative disease selected from Alzheimer's disease, corticobasal syndrome, Parkinson's disease, spinocerebellar degeneration, or amyotrophic lateral sclerosis.

The subject in present determination method 1, when determining the risk of onset of a disease caused by synaptic dysfunction or disease accompanied by synaptic dysfunction, can be generally a subject in which the disease caused by synaptic dysfunction or disease accompanied by synaptic dysfunction has not developed and it is unclear whether the disease caused by synaptic dysfunction or disease accompanied by synaptic dysfunction would develop in the future, and the subject in present determination method 1, when determining the presence or absence of onset of a disease caused by synaptic dysfunction or disease accompanied by synaptic dysfunction, can be generally a subject in which it is unclear whether the disease caused by synaptic dysfunction or disease accompanied by synaptic dysfunction has developed. Preferably, the subject has been evaluated (determined) as having a high risk of onset of a disease caused by synaptic dysfunction or disease accompanied by synaptic dysfunction by the method of determining the risk of onset of the invention. A control in present determination method 1 refers to a healthy subject who has no or hardly any risk of onset of a disease caused by synaptic dysfunction or disease accompanied by synaptic dysfunction and has not developed a disease caused by synaptic dysfunction or disease accompanied by synaptic dysfunction. To set the threshold value described above, one or more of (i) randomly selected healthy subjects or all healthy subjects who have already been subjected to determination of the concentration of DARPs, (ii) healthy subjects who are of the same age or age range as a subject (intracranial drebrin content decreases with age), and (iii) subjects who have not developed a disease, can be a control. Acute exacerbation stage of synaptic dysfunction may be distinguished by using a threshold value set from the concentration of DARPs of such controls.

Examples of the subject in present determination method 2 include subjects who have developed a disease caused by synaptic dysfunction or disease accompanied by synaptic dysfunction, but the severity of the disease caused by synaptic dysfunction or disease accompanied by synaptic dysfunction is unknown. For examples, the control in present determination method 2 refers to a patient who has developed a disease caused by synaptic dysfunction or disease accompanied by synaptic dysfunction and has the severity thereof determined. In this regard, a "patient who has the severity thereof determined" may be a patient who has the severity determined based on a known diagnostic guideline. It is desirable to use a diagnostic guideline for a target disease as the known diagnostic guideline. Examples of the known diagnostic guideline include a diagnostic guideline for dementia, diagnostic guideline for cerebral hemorrhage/cerebral infarction, etc. For example, dementia in Alzheimer's disease is categorized into early stage, intermediate stage, advanced stage, etc. by a medical interview, etc., and it is understood that plasma DARPs are at the maximum from the early stage to the intermediate stage of onset. In fact, it is demonstrated that DARPs rather decrease after widespread neuron death in the advanced stage (sever dementia patient) (patient AD01 in Example 2). In cerebral hemorrhage, it is also understood that DARPs would be high for a while after hemorrhage, and decrease after some time has passed in the chronic phase, and stabilize at a certain value. Since the relationship between the severity and change in DARPs of a control varies depending on the target disease in this manner, it is preferable to determine the severity from a suitable diagnostic guideline.

The synaptic dysfunction non-human animal in instant screening method 1 may be a non-human animal that has naturally developed synaptic dysfunction or a non-human animal caused to develop synaptic dysfunction. Specifically, a non-human animal caused to have synaptic dysfunction may be a non-human animal model of the disease caused by synaptic dysfunction or disease accompanied by synaptic dysfunction described above. Examples of the non-human animal described above include non-human mammals such as mice as well as rats, hamsters, guinea pigs, monkeys, cows, pigs, horses, rabbits, sheep, goats, cats, and dogs.

The cultured cell synaptic dysfunction model in instant screening method 2 may be a cultured cell that has naturally developed synaptic dysfunction or a cultured cell caused to have synaptic dysfunction. Specifically, a cultured cell caused to develop synaptic dysfunction may be a cultured cell model of the disease caused by synaptic dysfunction or disease accompanied by synaptic dysfunction described above. Preferred examples of the cultured cell include cultured neurons, cultured cells derived from humans, as well as cultured cells derived from non-human mammals such as mice rats, hamsters, guinea pigs, monkeys, cows, pigs, horses, rabbits, sheep, goats, cats, and dogs.

In one embodiment, the present invention relates to a kit for determining a risk of onset, the presence or absence of onset, or severity of a disease caused by synaptic dysfunction or disease accompanied by synaptic dysfunction, comprising the instant antibody (hereinafter, also referred to as the "instant kit"). In a preferred embodiment, the instant kit is a kit for use in the present determination methods. The instant kit may contain the instant antibody, as well as means for obtaining a biological sample from a subject, a labeled secondary antibody that recognizes the instant antibody, and a package insert describing the procedure or diagnostic standards.

While the present invention is more specifically described hereinafter with the Examples, the technical scope of the invention is not limited to the exemplification.

### Example 1

### 1. Detection of DARPs from a blood sample

The inventors conjectured that drebrin is present in the plasma of Alzheimer's disease patients, and performed Western blot on the plasma by using an anti-drebrin antibody. Meanwhile, drebrin could not be detected. In this regard, the sample was subjected to protein A/G bead treatment in order to remove antibody components contained in the plasma, and Western blot was performed.

The following is the experimental procedure.
(1) 1.8 ml of plasma and 200 µl of protein A/G beads (Protein A/G PLUS-Agarose: Santa Cruz Biotechnology) were suspended and reacted (preclearing) in a rotator for 1 hour at 4°C.
(2) The sample was centrifuged to prepare supernatant (preclear plasma) and precipitate (Beads Absorbed: beads absorbed fraction).
(3) 30 µl of SDS sample buffer was added to the beads absorbed fraction, which was thermally denatured and eluted.
(4) A part of the centrifuged supernatant (preclear plasma) was preserved for Input, and the rest of the preclear plasma and four types of antibodies (anti-drebrin anti-serum DEp: prepared by the inventors; anti-drebrin monoclonal antibodies 28D8 and 3D9 that recognize drebrin A and E: prepared by the inventors; control IgG: Fujifilm Wako Pure Chemical Corporation) were each mixed and reacted with o/n (IP: immunoprecipitation).
(5) 30 µl of protein A/G beads was further suspended in the IP tube of (4) reacted with o/n and reacted in a rotator for 1 hour at 4°C, then the beads were washed, and 30 µl of SDS sample buffer was added to the beads fraction, which was thermally denatured and eluted (IP, immunoprecipitation fraction).
(6) Each sample (top row: Beads absorbed, Plasma, Preclear Plasma; bottom row: each IP fraction) was analyzed by Western blot (a mixture of monoclonal antibodies 28D8 and 3D9 was used as the antibodies for detection).

The results are shown in Figure **1****.** Drebrin could not be detected by Western Blot in the bottom row where immunoprecipitation was performed, whereas a band of drebrin was unexpectedly found from a fraction of absorption of protein A/G beads (Beads absorbed) used in Preclear (top row, arrow). In this regard, to check whether said band is drebrin E that is present throughout the entire body or drebrin A that is localized in a synapse of the brain, Western blot was performed on the protein A/G beads absorbed fraction by using various anti-drebrin antibodies that recognize both drebrin A and E (M2F6, 28D8, 3D9, 22G5, 17C3, 33A4; prepared by the inventors) and anti-drebrin A antibodies that recognize a region comprising at least a part of Ins2 in drebrin A (4C2, prepared by the inventors; DAS2: Immuno-Biological Laboratories) . Meanwhile, the band of full length drebrin of about 130 kDa was not stained with drebrin A specific antibody 4C2 or DAS2. Thus, it was found that the band was a band of drebrin E (Figure **2**) . It is understood that said drebrin E is derived from renal tumor. Meanwhile, a plurality of bands understood to be a fragment or splice variant of drebrin A that is also stained by an anti-drebrin A antibody 4C2 or DAS2 were found on the side of lower molecular weight than the band of drebrin E. These were named drebrin A related proteins (DARPs). In Figure **2****,** drebrin E and DARP40 with a molecular weight of about 40 kDa among DARPs are indicated with an arrow. DARP40 was not clearly stained with control IgG or generic antibody of drebrin M2F6, and was discovered for the first time by the inventors. Since drebrin A is localized in dendritic spines of the brain, it was suggested that DARPs leak from a damaged dendritic spine in an Alzheimer's disease patient and migrate into blood through the blood-brain barrier.

### Example 2

### 2. Use of DARPs in blood sample as a diagnostic biomarker

It was understood that DARPs leak from damaged dendritic spines in an Alzheimer's disease patient in view of Example 1. DARPs can also leak from a damaged dendritic spine to migrate into blood in other diseases caused by synaptic dysfunction and diseases accompanied by synaptic dysfunction. In this regard, the following experiment was conducted to check whether DARPs in a blood sample can be used as a diagnostic biomarker for a disease caused by synaptic dysfunction or disease accompanied by synaptic dysfunction.

A blood sample was obtained from two Alzheimer's disease patients (ADO1 (5 years since onset, with renal tumor), ADO2 (3 years since onset, MMSE: 21 points)), one corticobasal syndrome patient (CBS), one Parkinson's disease patient (PD01), and a healthy 65 year old male (Healthy) and a protein A/G beads absorbed fraction was subjected to Western blot in the same manner as Example 1. Detection used an anti-drebrin A antibody DAS2 (Immuno-Biological Laboratories).

The results are shown in Figure **3****.** A large number of bands of DARPs were found from each of the blood samples. The DARPs that could be confirmed were named DARP40, DARP60, DARP70, DARP90, and DARP100 in accordance with the molecular weight. The concentration of DARPs was more elevated in blood samples derived from patients with a disease, except for AD01, relative to the healthy 65 year old male (Healthy). As shown in Figure **4****,** detection of DARPs by the same procedure using AD02 and control plasma (Ctrl: Kohjin Bio (KJB), normal human plasma/pool EDTA-2Na, lot: HMN389081) showed an increase in the concentration of DARP40, DARP60, DARP70, DARP90, and DARP100 in AD02. Thus, it was demonstrated that these DARPs can be used as a diagnostic biomarker for a disease caused by synaptic dysfunction or disease accompanied by synaptic dysfunction. Meanwhile, the concentration of DARPs was lower in AD01 than Healthy (Figure **3**). It is understood that a rapid leakage of DARPs much like in an acute exacerbation stage does not occur in AD patients in a chronic phase, where neuron death has occurred so that dementia has advanced to the final stage (high tau and low MMSE score), so that the concentration of DARPs is low.

Furthermore, a difference in the darkness of bands was not observed between the patients with a disease and the healthy 65 year old male (Healthy) when the same samples as Figure **3** were stained with an anti-drebrin antibody 3D9 that recognizes both drebrin A and E (prepared by the inventors) (Figure **5**). These results show that a drebrin related protein derived from drebrin E cannot be used as a diagnostic biomarker, and an antibody that recognizes a region comprising at least a part of Ins2 in drebrin A (DAS2, etc.) can be used for the diagnosis of diseases.

### Example 3

### 3. Use of DARPs in cerebrospinal fluid sample as a diagnostic biomarker

Example 2 was able to confirm that DARPs in a blood sample can be used as a diagnostic biomarker for a disease caused by synaptic dysfunction or disease accompanied by synaptic dysfunction. Since DARPs are related to drebrin A that is localized in the brain, it is understood that many DARPs are also present in a cerebrospinal fluid in patients of a disease caused by synaptic dysfunction or disease accompanied by synaptic dysfunction. In this regard, the following experiment was conducted to confirm that DARPs in a cerebrospinal fluid sample can be used as a diagnostic biomarker for a disease caused by synaptic dysfunction or disease accompanied by synaptic dysfunction.

A cerebrospinal fluid sample was obtained from three Alzheimer's disease patients (GHAD01 to 03, each having mild dementia), one amyotrophic lateral sclerosis patient (ALS01), one spinocerebellar degeneration patient (SCD01), and one depression patient (Dep01), and a protein A/G beads absorbed fraction was subjected to Western blot in the same manner as Example 2, except for using a cerebrospinal fluid sample instead of a blood sample. Detection used an anti-drebrin A antibody DAS2 (Immuno-Biological Laboratories).

The results are shown in Figure **6****.** A large number of bands of DARPs were found from each cerebrospinal fluid sample, and DARP40, DARP60, DARP70, DARP90, and DARP100 were observed just like in blood samples. The concentration of DARPs was higher in two Alzheimer's disease patients (GHAD01 and 03), one amyotrophic lateral sclerosis patient (ALS01), and one spinocerebellar degeneration patient (SCD01) than in one depression patient (Dep01), which are neurodegenerative diseases. There is also a difference in the concentrations of DARPs even between patients suffering from the same Alzheimer's disease as demonstrated from the comparison of GHAD01 and 03, suggesting that the concentration of DARPs can reflect the severity of the disease (degree of damage to a dendritic spine). Meanwhile, GHAD02 had the same level of concentration of DARPs as the one depression patient (Dep01). It is understood that the concentration of DARPs was low in GHAD02 because AD advanced and transitioned to the chronic phase, and a rapid leakage of DARPs as in an acute exacerbation stage has not occurred. While the concentration of DARPs in the depression patient was lower relative to patients of other neurodegenerative diseases, in view of the possibility of depression transitioning to a cognitive function disorder, it is understood that transition to a cognitive function disorder can be evaluated by an increase in the concentration of DARPs in depression patients.

### Example 4

### 4. Relationship between other indicators of Alzheimer's disease patients and the amount of DARPs

Example 3 demonstrated that DARPs in a cerebrospinal fluid sample can be used as a diagnostic biomarker for Alzheimer's disease. Meanwhile, it is understood that the concentration of DARPs is low because a rapid leakage of DARPs just like in an acute exacerbation stage has not occurred in patients who have transitioned to a chronic phase with the advancement of Alzheimer's disease. In this regard, DARPs in a cerebrospinal fluid sample were detected by Western blot in the same manner as Example 3 for the six

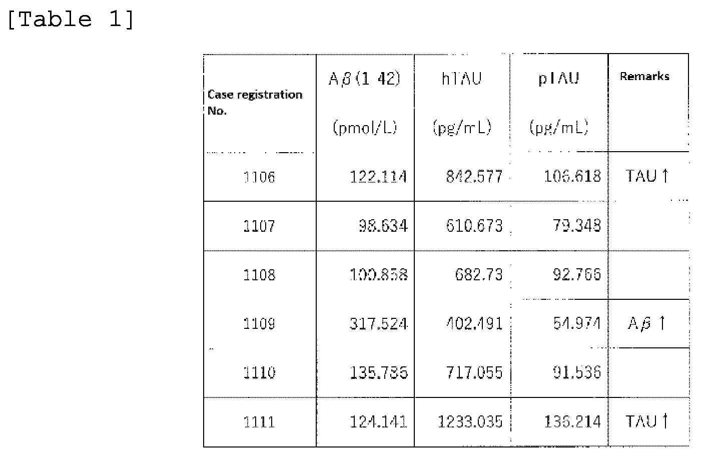

Alzheimer's disease patients described in the following Table 1. In the table, "TAU_{↑}" means that the concentration of tau is high to reach tau accumulation, and "Aβ_{↑}" means that the concentration of amyloid β is high to reach amyloid β accumulation.

The results are shown in Figure **7****.** DARP100, DARP70, and DARP40 were found from a cerebrospinal fluid sample from all patients. While the concentration of DARPs was high in patients in an exacerbation stage not reaching tau accumulation (case nos. 1107 to 1110), the concentration of DARPs was low in the patient of case registration no. 1111 with the highest tau. This result shows that the concentration of DARPs is low in a patient who has transitioned to a chronic stage with the advancement of Alzheimer's disease.

### Example 5

### 5. Validation of neurocytotoxicity and confirmation of the presence of DARP autoantibodies

Example 1 demonstrated that DARPs adsorb onto protein A/G beads with affinity to an antibody (IgG, IgA). To check whether such adsorption is direct adsorption of DARPs to protein A/G beads or through a DARP autoantibody in a blood sample, GFP tag added human drebrin A (GFP-hDA: sample expressed in HEK293 and homogenized with SDS buffer) was subjected to SDS-PAGE, and an anti-drebrin antibody (3D9: prepared by the inventors) and plasma from an Alzheimer's disease patient (AD plasma) were used as a primary antibody for detection.

The results are shown in Figure **8****.** A band was found at the same position (arrow in the figure) between detection with 3D9 on the left side of Figure **8** and detection with plasma from an Alzheimer's disease patient (AD plasma) on the right side of Figure **8****.** This result revealed that an autoantibody which can recognize drebrin A is present in plasma from an Alzheimer's disease patient. It is understood that DARPs adsorbed onto protein A/G beads via the autoantibody.

Since DARPs of a cerebrospinal fluid also adsorb onto protein A/G beads, it is understood that an anti-DARP autoantibody is also present in a cerebrospinal fluid. In this regard, to check whether an anti-DARP autoantibody can be toxic to brain neurons, an anti-drebrin antibody (22G5: prepared by the inventors) or commercially available normal mouse IgG (Fujifilm Wako Pure Chemical Corporation) was administered to a DIV23 (number of days of culture in vitro) cultured rat hippocampal cell (prepared by the inventors). The cell was incubated for 1 hour, washed with PBS, and immobilized, then reacted with an anti-MAP2 antibody (Abeam). 22G5 and normal mouse IgG were made visible with an anti-mouse IgG-Alexa 568 (Thermo Fisher Scientific), and MAP2 was made visible with anti-rabbit IgG-Alexa 488 (Thermo Fisher Scientific) to examine the effect of an anti-drebrin antibody on neurons.

The results are shown in Figure **9****.** For the cultured rat hippocampal cell that was administered with commercially available normal mouse IgG (Fujifilm Wako Pure Chemical Corporation) for one hour, washed, and immobilized, mouse IgG (red; not detected) was not taken up into the cell, and the same MAP2 staining (green) as normal cells was observed (Figure **9B**). Meanwhile, an image (red) of 22G5 being incorporated into soma or some dendrites was observed in cultured neurons that were administered with an anti-drebrin antibody (22G5) for 1 hour, washed, and immobilized. Furthermore, the area where 22G5 was incorporated had an abnormality in microtubules and lost normal MAP2 staining (green) (Figure **9A**). This result suggests that production of a large amount of DARP autoantibodies in the body due to development of a disease caused by synaptic dysfunction or disease accompanied by synaptic dysfunction damages neurons in the brain and further advances the pathological condition. Thus, it is understood that a dominant negative peptide, which suppresses the effect of a DARP autoantibody damaging brain neurons, would be a candidate compound for a therapeutic agent and/or prophylactic agent for the disease described above that can suppress damage to brain neurons.

### Example 6

### 6. Checking the presence of DARPs in rat neurons

In order to check whether an animal disease model or cultured neurons derived from an animal disease model can be used in screening for a therapeutic agent or prophylactic agent for a disease, the presence of DARPs in rat neurons was examined by the following procedure.
(1) DIV8 (number of days of culture in vitro) cultured rat cerebral cortical cells for 2 dishes (60 mm dish) were collected with PBS and sonicated to disrupt the cells.
(2) After the samples were centrifuged for 20 minutes at 15000 rpm, the supernatant was collected (the left lane in Figure **10****,** Input).
(3) For immunoprecipitation, an anti-drebrin antibody (28D8: prepared by the inventors) and 30 µl of protein A/G beads were added to the supernatant, which was rotated (one hour), then the beads were washed three times with PBS (PBS was added and admixed, and the mixture was centrifuged for 10 seconds at 1500g to remove the supernatant), and the substance bound was eluted with 50 µl of SDS buffer (center lane in Figure **10****,** 28D8 IP).
(4) As a negative control, 30 µl of protein A/G beads was added to supernatant, which was rotated (one hour), and then the beads were washed three times with PBS (PBS was added and admixed, and the mixture was centrifuged for 10 seconds at 1500g to remove the supernatant), and the substance bound was eluted with 50 µl of SDS buffer (right lane in Figure **10****,** A/G beads).
(5) Each of the resulting fractions was subjected to Western blot. Detection used an anti-drebrin A antibody DAS2 (Immuno-Biological Laboratories).

The results are shown in Figure **10****.** A band of DARPs was observed in a fraction subjected to immunoprecipitation with an anti-drebrin antibody. It was revealed that DARPs are also present in animals with drebrin other than humans and cultured cells thereof. This result suggests that a change in the concentration of DARPs which leak out from a cell (into culture) due to synaptic dysfunction can be used as an indicator in screening for a prophylactic agent or a therapeutic agent for a disease caused by synaptic dysfunction or disease accompanied by synaptic dysfunction.

### Example 7

### 7. Examination of the presence of DARPs in a mouse Alzheimer's disease model

The inventors have reported a heterologous mouse having a 5x FAD gene and drebrin gene (+/-) (5x FAD/DXKO^{+/-}) by crossing a 5x FAD mouse (mouse with 5 genes of familial Alzheimer's disease) and a drebrin knockout mouse (homo) (Japanese Patent Application No. 2019-185245). A blood sample was obtained from this mouse, and a protein A/G beads absorbed fraction was subjected to Western blot in the same manner as Example 1. When detection was performed using an anti-drebrin A antibody DAS2 (Immuno-Biological Laboratories), it was confirmed that the DARP100 concentration of 5x FAD/DXKO^{+/-} was higher than a wild-type mouse.

### [Industrial Applicability]

Since the presence or absence of development or severity of a disease caused by synaptic dysfunction or disease accompanied by synaptic dysfunction, which depended on a Q&A test (depression, dementia, etc.) or image test (cerebral hemorrhage, etc.) in the past, can be determined in the early stages in a simple manner with the determination method of the invention, such a method leads to prevention of development of such diseases and to treatment in the early stages. Furthermore, DARPs and anti-DARP autoantibodies can be an indicator for screening for a prophylactic agent or therapeutic agent and contribute to research for drug development.

Thus, the industrial applicability of the invention in the medical/pharmaceutical fields is very high.

## Claims

1. A method of determining a risk of onset, or the presence or absence of onset of, a disease caused by synaptic dysfunction or disease accompanied by synaptic dysfunction, comprising steps (a-1) to (c-1):
(a-1) a step for measuring a concentration of drebrin A related proteins (DARPs) in a biological sample obtained from a subject;
(b-1) a step for comparing the concentration of DARPs measured in step (a-1) with a concentration of DARPs in a control who does not develop a disease caused by synaptic dysfunction or disease accompanied by synaptic dysfunction; and
(c-1) a step for evaluating the subject as having a high risk of onset of a disease caused by synaptic dysfunction or disease accompanied by synaptic dysfunction or as highly likely to have developed a disease caused by synaptic dysfunction or disease accompanied by synaptic dysfunction if the concentration of DARPs measured in step (a-1) is higher than the concentration of DARPs in the control.

2. A method of determining a severity of a disease caused by synaptic dysfunction or disease accompanied by synaptic dysfunction, comprising steps (a-2) to (c-2):
(a-2) a step for measuring a concentration of drebrin A related proteins (DARPs) in a biological sample harvested from a subject who has developed a disease caused by synaptic dysfunction or disease accompanied by synaptic dysfunction;
(b-2) a step for comparing the concentration of DARPs measured in step (a-2) with a concentration of DARPs in a control who has developed a disease caused by synaptic dysfunction or disease accompanied by synaptic dysfunction; and
(c-2) a step for evaluating the subject as highly likely to have a more severe disease caused by synaptic dysfunction or disease accompanied by synaptic dysfunction than the control if the concentration of DARPs and/or DARP autoantibodies measured in step (a-2) is higher than the concentration of DARPs and/or DARP autoantibodies in the control.

3. A method of screening for a prophylactic agent or a therapeutic agent for a disease caused by synaptic dysfunction or disease accompanied by synaptic dysfunction, comprising steps (a-3) to (c-3):
(a-3) a step for measuring a concentration of drebrin A related proteins (DARPs) in a sample harvested from a non-human animal synaptic dysfunction model administered with a test substance;
(b-3) a step for comparing the concentration of drebrin A related proteins (DARPs) measured in step (a-3) with a concentration of drebrin A related proteins (DARPs) in a non-human animal synaptic dysfunction model which is not administered with the test substance; and
(c-3) a step for evaluating the test substance as effective in prophylaxis or treatment of a disease caused by synaptic dysfunction or disease accompanied by synaptic dysfunction if the concentration of drebrin A related proteins (DARPs) measured in step (a-3) is lower than the concentration of drebrin A related proteins (DARPs) in the non-human animal synaptic dysfunction model which is not administered with the test substance.

4. A method of screening for a prophylactic agent or a therapeutic agent for a disease caused by synaptic dysfunction or disease accompanied by synaptic dysfunction, comprising steps (a-4) to (d-4):
(a-4) a step for administering a test substance to a cultured cell synaptic dysfunction model and culturing the model;
(b-4) a step for measuring a concentration of drebrin A related proteins (DARPs) in a culture of the cultured cell synaptic dysfunction model;
(c-4) a step for comparing the concentration of DARPs measured in step (b-4) with a concentration of DARPs in a culture of a cultured cell synaptic dysfunction model which is not administered with the test substance; and
(d-4) a step for evaluating the test substance as effective in prophylaxis or treatment of a disease caused by synaptic dysfunction or disease accompanied by synaptic dysfunction if the concentration of DARPs measured in step (b-4) is lower than the concentration of DARPs in the culture of a cultured cell synaptic dysfunction model which is not administered with the test substance.

5. The method of claims 1 to 3, wherein the biological sample is a cerebrospinal fluid sample or a blood sample.

6. The method of claims 1 to 5, wherein the DARPs are one or more types selected from DARP40, DARP60, DARP70, DARP90, and DARP100.

7. The method of claims 1 to 6, wherein the disease caused by synaptic dysfunction or disease accompanied by synaptic dysfunction is a neurodegenerative disease.

8. The method of claim 7, wherein the neurodegenerative disease is selected from Alzheimer's disease, corticobasal syndrome, Parkinson's disease, spinocerebellar degeneration, and amyotrophic lateral sclerosis.

9. The method of claim 1 to 8, wherein the concentration of DARPs is measured by using an antibody that recognizes a drebrin A specific epitope.

10. An antibody for use in the method of claim 9, **characterized by** recognizing a drebrin A specific epitope.

11. A kit for determining a risk of onset, the presence or absence of onset, or severity of a disease caused by synaptic dysfunction or disease accompanied by synaptic dysfunction, comprising the antibody of claim 10.

12. A method of screening for a prophylactic agent or a therapeutic agent for a disease caused by synaptic dysfunction or disease accompanied by synaptic dysfunction, **characterized by** searching for a dominant negative peptide to a drebrin A related protein (DARP) autoantibody.
